(19) Europäisches Patentamt
European Patent Office
Office européen des brevets

(11) Publication number: **0 243 125 B1**

(12) **EUROPEAN PATENT SPECIFICATION**

(45) Date of publication of patent specification: **12.08.92**

(51) Int. Cl.5: **C12Q 1/00**, //C12Q1/42, C12Q1/56,C12Q1/28

(21) Application number: **87303432.6**

(22) Date of filing: **16.04.87**

(54) **Method of measuring phenol and alpha-naphthol, and method of measuring the activity of enzymes.**

(30) Priority: **19.04.86 JP 89201/86**
**19.04.86 JP 89202/86**

(43) Date of publication of application:
**28.10.87 Bulletin 87/44**

(45) Publication of the grant of the patent:
**12.08.92 Bulletin 92/33**

(84) Designated Contracting States:
**CH DE FR GB IT LI**

(56) References cited:
**EP-A- 0 033 539**
**EP-A- 0 054 358**
**EP-A- 0 123 902**
**EP-A- 0 148 275**
**DE-A- 3 213 786**
**GB-A- 2 095 401**
**US-A- 4 378 429**
**US-A- 4 427 770**

(73) Proprietor: **SANWA KAGAKU KENKYUSHO CO., LTD.**
**No. 35, Higashi-sotobori-cho**
**Higashi-ku Nagoya-shi Aichi-ken(JP)**

(72) Inventor: **Uno, Shizuo**
**2-116-2, Fujigaoka**
**Kasugai-shi Aichi-ken(JP)**
Inventor: **Kurono, Masayasu**
**3-6-7, Sasao-Nishi Toin-cho**
**Inabe-gun Mie-ken(JP)**
Inventor: **Katsumoto, Keiko 2-418,**
**Kamiyashiro Kita-juhtaku**
**1-202, Yashiro-guchi Meito-ku**
**Nagoya-shi Aichi-ken(JP)**
Inventor: **Asai, Noboru**
**2296, Mabiki Yamato-cho**
**Ichinomiya-shi Aichi-ken(JP)**
Inventor: **Sawai, Kiichi**
**1-36-14, Ninomiya**
**Funabashi-shi Chiba-ken(JP)**

(74) Representative: **Diamond, Bryan Clive et al**
**Gee & Co., Chancery House, Chancery Lane**
**London WC2A 1OU(GB)**

EP 0 243 125 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a highly sensitive method of measuring phenol or α-naphthol as well as a method of measuring the activity of an enzyme through the measurement of phenol or α-naphthol.

The measurement of activity of alkaline phosphase, acid phosphatase, kallikrein, antithrombin III and the like enzymes is quite important in clinical diagnosis of various diseases and has already been widely utilized therefor.

Each of the measurements has been carried out through a measurement of phenol or α-naphthol which is liberated by contacting the enzyme with a substrate of a phenyl or naphthyl radical containing compound. Although various methods have been reported for measuring phenol and α-naphthol, typical ones are listed as follows.

Qualitative Methods using Phenol:

Detection through indophenol reaction (Liebermann's reaction); Detection with Millon's reagent; Detection with 2,6-dichloroquinonchloroimide; Detection with 4-aminoantipyrine; Detection with iron(III) chloride; Detection through fluorescein reaction of m-diphenol.

Quantitative Methods of Phenol:

Method utilizing diazo-coupling; Method using 2,6-dibromoquinonchloroimide; Method using 4-aminoantipyrine; Method using Folin-Ciocalten's reagent.

Qualitative Methods of α-naphthol:

Detection with diazonium salt of p-nitroaniline; Detection with cerium ammonium nitrate.

Quantitative Methods of α-naphthol:

Method using 2-naphthylamine-5,7-disulfonic acid; Method using 4-aminoantipyrine.

Almost all of those methods, especially the quantitative methods, have a common disadvantage in that operational procedures are troublesome so as to require a relatively long period of time. The most widely employed methods amongst them, in view of their ease of operation, are the qualitative and quantitative methods using 4-aminoantipyrine which utilize, as far as phenol is concerned, a reaction between phenol and 4-aminoantipyrine under alkaline condition and in the presence of potassium ferricyanide, for forming an antipyrine dye of yellowish red color to detect the phenol, and a result of measurement of color tone of the resulting colored solution through a measurement of absorbancy at 510nm to quantitively determine the phenol, as shown below by reaction formulae.

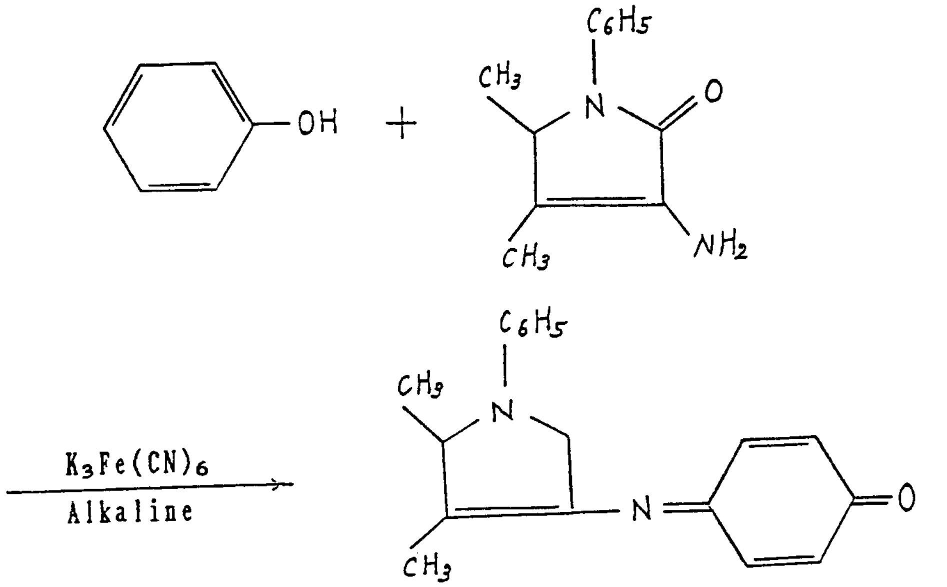


(Antipyrine dye)

The methods using 4-aminoantipyrine, which have been preferable, have also the disadvantage of low sensitivity, since the antipyrine dye to be formed has a lower molecular extinction coefficient of 5000 to 10000. Therefore, if a concentration of phenol or α-naphthol in a sample is relatively low, the formed antipyrine dye should be extracted with chloroform and concentrated to increase the sensitivity, which make the operation troublesome and requires an additional period of time therefor.

As a conventional method for measuring the activity of enzymes, for instance alkaline phosphatase and acidic phosphatase, the Kind-King's method has widely been employed. According to this method, the enzyme is contacted with a substrate of a phenyl or naphthyl radical containing compound to make phenol or α-naphthol free from the substrate, reacting the free phenol or α-naphthol with 4-aminoantipyrine in the presence of potassium ferricyanide as an oxidant to form the antipyrine dye with yellowish red color, and measuring a color tone of the resulting colored solution to determine an activity of the enzyme.

This method has the disadvantages as referred to in the measurement of phenol. Further, this method shows a remarkable disadvantage in that a maximum absorption of the antipyrine dye lies near 500nm as said above and thus such wave-length is employed for the measurement. Namely, when the activity of enzymes is measured in the field of clinical examinations, a serum is generally selected as a test sample and there is a possible abnormal one of a hemolytic, chylous or icteric serum, and in case of such abnormal samples, haemoglobin, chyme or bilirubin acts as disturbing material to cause an error in measurement, since a maximum absorption of the materials overlaps with or near 500nm as the measuring wave-length for the antipyrine dye, as follows.

Haemoglobin : having dual maximum absorptions at 430nm and range of 530 to 570nm,

Chyme : absorbing over a wide wave-length range and showing a higher absorbancy, as a measuring wave-length becomes shorter, and

Bilirubin : having a maximum absorption at 465nm.

An object of the invention is to provide a highly sensitive method for measuring phenol and α-naphthol, which requires a relatively short period of time for reaction therefor.

Another object of the invention is to provide a method for measuring the activity of enzymes through the measurement of phenol or α-naphthol, which requires relatively short period of time and shows a high accuracy, even if an abnormal serum will be employed as an enzyme containing sample to be tested.

According to the invention we provide a method of quantitatively measuring phenol or α-naphthol contained in a sample, which comprises reacting the phenol or α-naphthol with p-diethylaminoaniline in the presence of a chemical oxidizing agent to form a quinonic dye, and measuring the intensity of color of the resulting solution to determine the amount of the phenol or -naphthol.

As the oxidant, potassium ferricyanide, chloramine T, sodium metaperiodate or the like chemical oxidizing agent may be employed.

The invention also provides a method of measuring an activity of enzymes contained in a sample, which

comprises contacting a compound containing a phenyl or naphthyl radical with the enzyme in the sample to liberate the phenol or α-naphthol, reacting the free phenols or α-naphthol with p-diethylaminoaniline in the present of a chemical oxidizing agent to form a quinonic dye, and measuring the intensity of color of the resulting solution to determine the activity of the enzyme.

As the enzyme to have its activity measured, examples are alkaline phosphatase, acidic phosphatase, kallikrein, and antithrombin III. As the substrate, conventional compounds having the ability of liberating phenol and α-naphthol can be employed, for instance disodium and L-prolyl-L-phenylalanyl-L-alginine-1-naphthyl ester dihydrochloride are such compounds.

The following equations illustrate the reactions occurring in the above methods of the invention, in which the following substances are employed.

Enzyme, activity of which is measured : Alkaline phosphatase (ALP)
Substrate : Phenyl phosphate

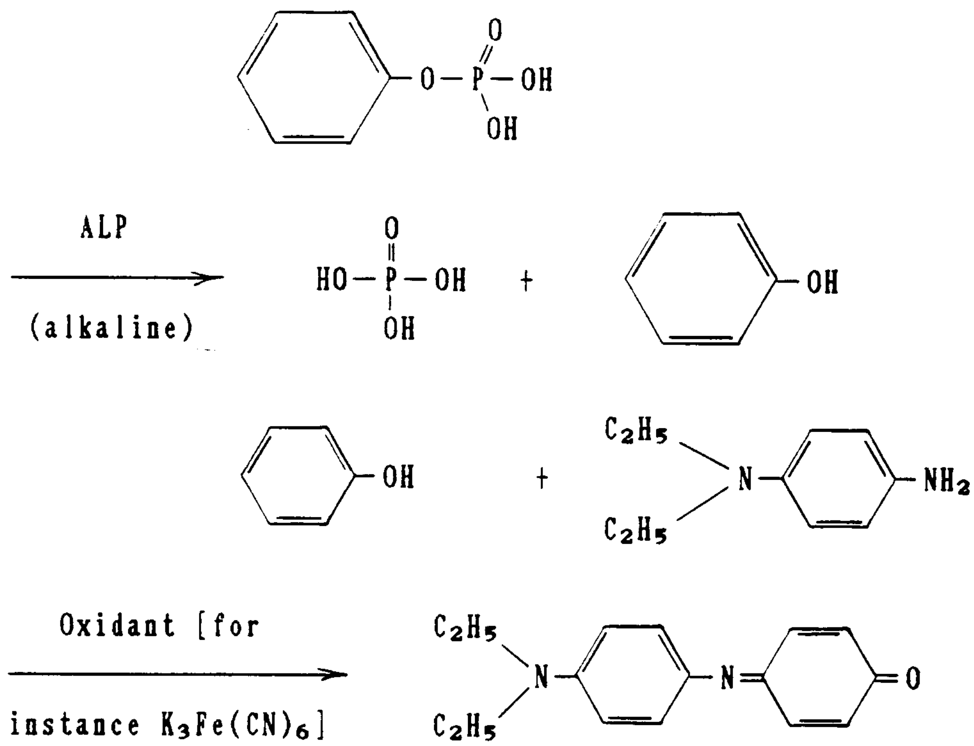


(Blue colored quinonic dye)

The resulting quinonic dye has its maximum absorption near 670 nm and thus such a high wave-length can be employed for the measurement.

Therefore, even if an abnormal serum is employed as the sample, the disturbances caused by haemoglobin and bilirubin can be avoided, due to differences in maximum absorption and the disturbance caused by chyme can also be inhibited to a low level. Further, the quinonic dye has a molecular extinction coefficient of about 20,000, which is about 2 to 4 times greater than that of the antipyrine dye (about 5000 to 10000) measured in the conventional Kind-King's method, so that the method of the present invention shows a quite high sensitivity, and each of the enzyme and oxidizing reactions is completed in about 5 minutes which is about one third of the time required in the conventional Kind-King's method.

The invention will now be explained with reference to test Examples and measuring Examples, which may refer to the drawings in which:

Fig. 1 is a graph showing the relation between absorbency and measuring wave-length on sample solution containing a quinonic dye and a control solution;

Fig. 2 is a calibration curve to be employed when phenol is measured according to the invention;

Fig. 3 is a graph showing the effect of hemoglobin added to a normal serum on the measurement of the activity of alkaline phosphatase;

Fig. 4 is a graph similar to Fig. 3 but to test the effect of bilirubin;

Fig. 5 is a graph similar to Fig. 3, but to test the effect of chyme;
Fig. 6 is a graph similar to Fig. 3, but to test the effect of ascorbic acid; and
Fig. 7 is a graph showing the correlation between the method of the present invention and the conventional Kind-King's method.

Test Example 1

a) Test sample:
A blue colored aqueous solution containing a quinone dye which was prepared by reacting phenol with p-diethylaminoaniline as a diamine compound, in the presence of potassium ferricyanide, as an oxidant.
b) Control sample:
An aqueous solution containing the diamine compound and oxidant as in said test sample but not including phenol.
c) Measurements of absorbancy and molecular extinction coefficient:
The absorbancy of each of the test and control samples were measured at various wave-lengths to obtain results shown in Fig. 1. From the figure, it is apparent that a maximum absorption of the quinone dye in the test sample lies near 670 nm. Further, this quinone dye showed a molecular extinction coefficient of about 20000, which value is 2 to 4 times of that on the antipyrine dye measured in the conventional Kind-King's method. This shows that the method according to the invention has a sensitivity superior to that of the Kind-King's method.

Example 1 (Measurement of Phenol)

[I] Reagents

1) Carbonate buffer:
An aqueous solution prepared by mixing 50 mmol/l sodium carbonate solution with 50 mmol/l sodium hydrogen carbonate solution and adjusting the pH of the mixture to 10.0.
2) Potassium ferricyanide:
An aqueous solution of 30 mmol/l potassium ferricyanide solution.
3) p-Diethylaminoaniline:
An aqueous solution prepared by adding p-diethylaminoaniline dihydrochloride into 0.1 N hydrochloric acid solution, so that the concentration of p-diethylaminoaniline was made 15 mmol/l.

[II] Operational procedure

(1) Measuring operations:
a) 40 $\mu$l of each of a sample solution (T), a reagent blank as a control and a standard phenol solution (St) were taken.
b) To each solution taken as above, 2.0 ml of the carbonate buffer, 0.2 ml of potassium ferricyanide solution and 0.2 ml of a p-diethylaminoaniline solution were added and mixed, and then each of the resulting mixtures was left to stand for 5 minutes.
c) The absorbancy of the mixed solution containing sample and the mixed solution containing the standard phenol solution was measured at 670 nm (absorbancy values of Et and Es), while comparing same with the absorbancy of the reagent blank as control.
(2) Quantitative calculation:
A concentration (C) of phenol in the sample solution can be calculated in accordance with the following equation.

C = (Et/Es) x (concentration of standard phenol solution)

The volume ratio between the sample solution and reagents may be modified depending on the purpose of the measurement, for instance a purity measurement of raw material for drugs, such as propanol hydrochloride or a measurement of phenol in a waste solution from a factory, and taking the concentration of finally reacted solution into consideration.

Test Example 2 (Calibration Curve for Phenol)

The relation between the amount of phenol and absorbancy of the solution was determined on various standard phenol solutions containing phenol in various amounts ranging from 0 to 30 $\mu$g, by reacting phenol with reagents similar to that as described in Example 1, to obtain the results shown in Fig. 2. Both have a linear relation, which means the graph of Fig. 2 can be used as a calibration curve.

Example 2 (Measurement of activity on alkaline phosphatase)

[I] Reagents

1) First reagent:

To 100 ml of 50 mmol/l carbonate buffer (pH 10.0) containing 0.1% of polyoxyethylene octylphenylether, 380 mg of disodium phenyl phosphate, 100 mg of potassium ferricyanide and 100 mg of magnesium chloride (hexahydrate) were added and dissolved to prepare this reagent. This reagent can be effectively used over one month, if stored in a refrigerator.

2) Second reagent:

This reagent was prepared by adding and dissolving 36 mg of p-diethylaminoaniline dihydrochloride into 1 ml of 1N-hydrochloric acid and adding refined water to make the total amount to 20 ml.

[II] Measuring method

1) Measuring operations:

a) 2.0 ml of the first reagent were taken into each test tube and warmed at 37°C.

b) 40 $\mu$l of each of sample serum (T), refined water as control and a standard serum (St) were taken and added to each of said test tubes and warmed at 37°C for 5 minutes to cause a reaction.

c) Both of the reacted solutions were subjected to measurement of absorbancy at 660 nm, while comparing same with the refined water as a control, to obtain absorbancy values $Eo_{(T)}$ and $EO_{(St)}$.

d) Immediately after having made the absorbancy measurement, 0.4 ml of the second reagent were added to the reacted solution, mixed therewith and left to stand for 5 minutes to cause a reaction.

e) Both of the resulting solutions were subjected to measurement of absorbancy of 660 nm, while comparing the same with the refined water as control, to obtain absorbancy values $Ef_{(T)}$ and $Ef_{(St)}$.

2) Quantitative calculation

The activity (C) of alkaline phosphatase in the sample serum is calculated in accordance with the following equation:

$$C = \frac{Ef_{(T)} - [Eo_{(T)} \times (2.04/2.44)]}{Ef_{(St)} - [Eo_{(St)} \times (2.04/2.44)]} \times \text{(activity of the standard serum)}$$

Example 3 (Measurement of activity on urine kallikrein)

[I] Reagents

1) Test piece:

A reagent was prepared by dissolving 10 mg of p-diethylaminoaniline dihydrochloride and 6 mg of Pro-Phe-Arg-ONap• 2HCl (L-prolyl-L-phenylalanyl-L-alginine-1-naphthyl ester dihydrochloride) into 10 ml of 0.2 M phosphate buffer (pH 6.5). A filter paper is dipped into the reagent solution, taken out therefrom and freeze-dried under vacuo.

The treated test paper was cut into pieces having a size of 5 x 5 mmol/l and each piece was attached to an end of a polyvinyl chloride film strip with the aid of another piece having an adhesive layer on both sides, to prepare a desired test piece.

2) Color developing solution:

6 mmol/l Chloramine T solution.

[II] Measuring method

a) The test paper part of the test piece was dipped into a urine sample or one drop of the urine sample was dropped on the test paper part to cause absorption therein and then gently wiped on an upper surface of the test paper.
b) The resulting test piece was placed on a plate kept at 37° C and left as it was for 5 minutes.
c) One drop of the color developing solution was dropped on the test paper part of the test piece and left to stand for 2 minutes.
d) A blue color was developed on the test paper part and its color tone was compared with a previously prepared standard colorimetric chart [for instance, showing color tones which correspond to urine kallikrein concentrations of 0, 20, 40 and 80 NU/l (NU : naphthyl unit)] to determine the activity of kallikrein in the urine sample.

Test Example 3 (Influence of Disturbing substances)

Tests were carried out in connection with measurement of activity on alkaline phosphatase to determine whether or not hemoglobin, bilirubin, chyme and ascorbic acid exert a bad influence on the method of the invention which is carried out through the measurement of the quinone dye, since the substances have been considered as causes of a measuring error in the conventional Kind-King's method. Results are shown in Figs. 3 to 6. As is apparent from the figures, these substances exert almost no influence on the measurement.

Test Example 4 (Reproducibility)

The reproducibility of the method of the invention was checked to obtain the results given in the following Table. As can be seen from the Table, the invention method has a quite high reproducibility.

| | Serum 1 | Serum 2 |
|---|---|---|
| | (K -A unit) | |
| | 8.5 | 20.5 |
| | 8.3 | 21.0 |
| | 8.6 | 20.8 |
| | 8.4 | 20.9 |
| | 8.2 | 21.0 |
| | 8.6 | 21.0 |
| | 8.1 | 20.9 |
| | 8.4 | 21.2 |
| | 8.7 | 21.1 |
| | 8.4 | 21.2 |
| Mean value | 8.42 | 20.96 |
| Coefficient of variation (%) | 2.2 | 0.99 |

Test Example 5 (Correlation with Kind-King's method)

The activity of alkaline phosphatases in various samples was measured in accordance with the present invention method and conventional Kind-King's method, respectively to check the correlation therebetween. As seen from results shown in Fig. 7, both methods have a good correlation, which shows the reliability of the method of the invention.

**Claims**

**1.** A method of quantitatively measuring phenol or α-naphthol contained in a sample, which comprises reacting the phenol or α-naphthol with p-diethylaminoaniline in the presence of a chemical oxidizing agent to form a quinonic dye, and measuring the intensity of color of the resulting solution to determine the amount of the phenol or α-naphthol.

2. A method of measuring the activity of enzymes contained in a sample, which comprises contacting a compound containing a phenyl or naphthyl radical with the enzyme in the sample to liberate the phenol or α-naphthol, reacting the free phenol or α-naphthol with p-diethylaminoaniline in the present of a chemical oxidizing agent to form a quinonic dye, and measuring the intensity of color of the resulting solution to determine the activity of the enzyme.

3. A method as claimed in Claim 2, wherein the enzyme is alkaline phosphatase.

4. A method as claimed in Claim 1, 2 or 3, wherein the oxidizing agent is potassium ferricyanide, chloramine T or sodium metaperiodate.

**Revendications**

1. Méthode pour mesurer quantitativement le phénol ou le α-naphthol contenu dans un échantillon, qui comprend les étapes consistant à mettre en réaction le phénol ou le α-naphthol avec de la p-diéthylaminoaniline en présence d'un agent d'oxydation chimique pour former un colorant quinonique, et à mesurer l'intensité de couleur de la solution qui en résulte afin de déterminer la teneur en phénol ou α-naphthol.

2. Méthode pour mesurer l'activité des enzymes contenus dans un échantillon, qui comprend les étapes consistant à mettre en contact un composé contenant un radical phényle ou naphthyle avec l'enzyme dans l'échantillon pour libérer le phénol ou le α-naphthol, à faire réagir le phénol ou le α-naphthol libre avec la p-diéthylaminoaniline en présence d'un agent d'oxydation chimique pour former un agent quinonique et à mesurer l'intensité de couleur de la solution qui en résulte afin de déterminer l'activité de l'enzyme.

3. Méthode selon la revendication 2, dans laquelle l'enzyme est du phosphatase alcalin.

4. Méthode selon la revendication 1, 2 ou 3, dans laquelle l'agent d'oxydation est du ferricyanure de potassium, de la cloramine T ou du métapériodate de sodium.

**Patentansprüche**

1. Verfahren zur quantitavien Messung von Phenol oder α-Naphthol, die in einer Probe enthalten sind, umfassend die Umsetzung des Phenols oder des α-Naphthols mit p-Diethylaminoanilin in Gegenwart eines chemischen Oxidationsmittels zur Bildung eines Chinonfarbstoffes und Messung der Farbintensität der resultierenden Lösung, um die Menge des Phenols oder α-Naphthols zu bestimmen.

2. Verfahren zur Messung der Aktivität von Enzymen, die in einer Probe enthalten sind, enthaltend das Inkontaktbringen einer Verbindung, welche ein Phenyl- oder Naphthylradikal enthält mit dem Enzym in der Probe um das Phenyl oder α-Naphthol freizusetzen, Umsetzen des freien Phenols oder α-Naphthols mit p-Diethylaminoanilin in Gegenwart eines chemischen Oxidationsmittels um einen Chinonfarbstoff zu bilden und Messen der Farbintensität der resultierenden Lösung um die Enzymaktivität zu bestimmen.

3. Verfahren gemäss Anspruch 2, worin das Enzym alkalische Phosphatase ist.

4. Verfahren gemäss Anspruch 1, 2 oder 3, worin das Oxidationsmittel Kaliumferricyanid, Chloramin T oder Natriummetaperiodat ist.

FIG. 1

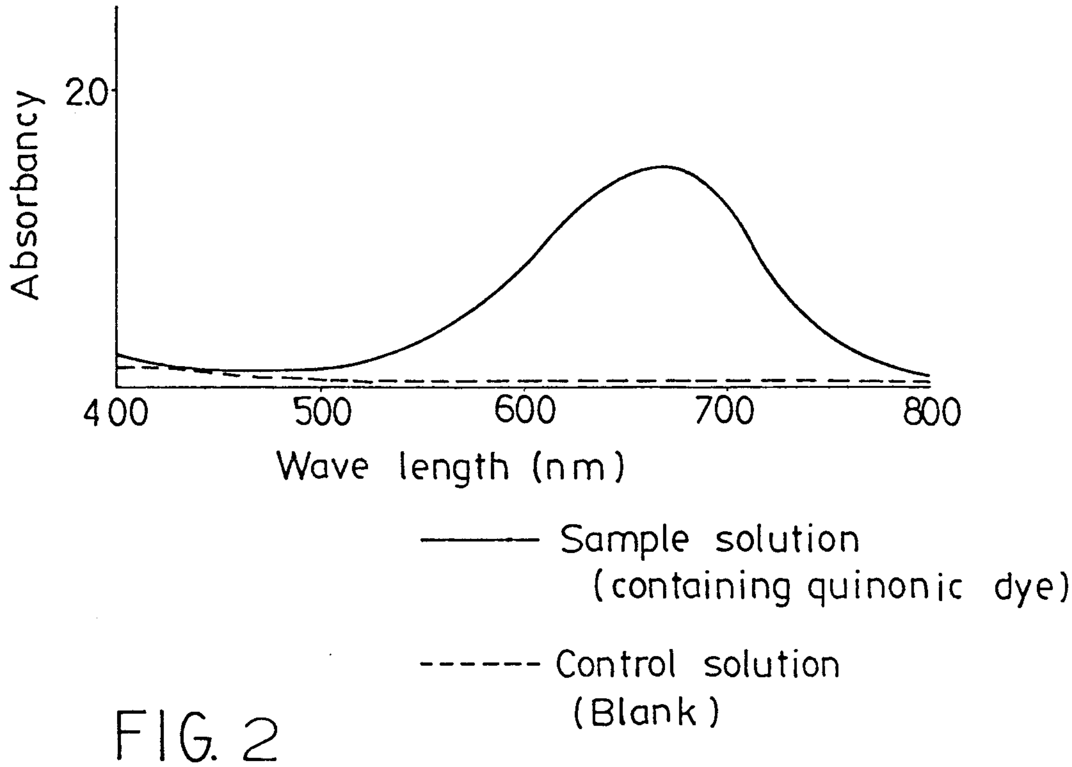
Absorbancy
2.0
400
500
600
700
800
Wave length (nm)
Sample solution
(containing quinonic dye)
Control solution
(Blank)

FIG. 2

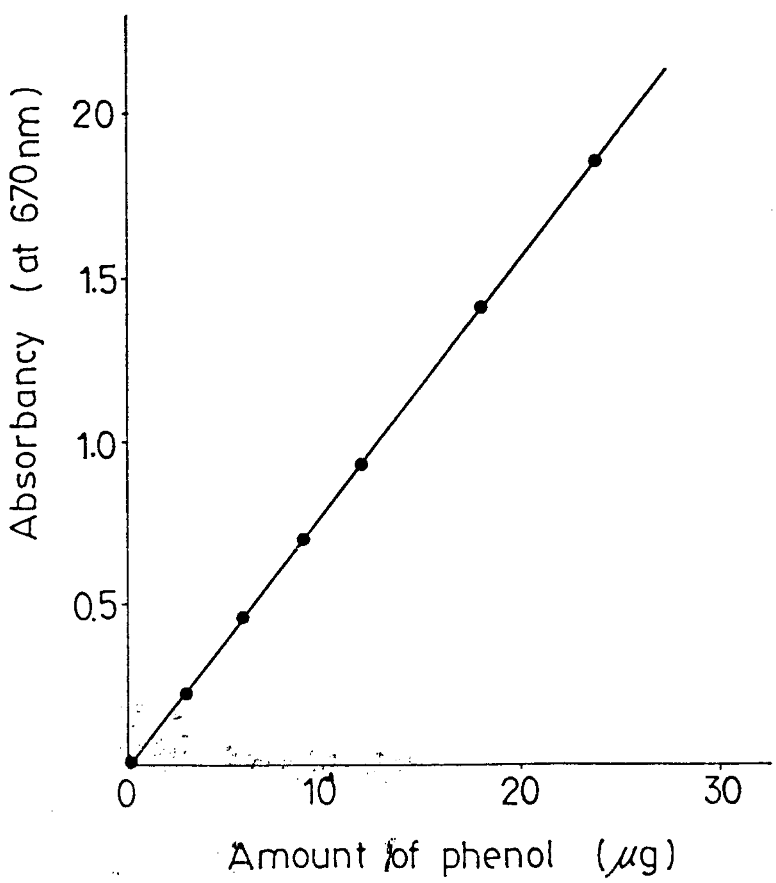
Absorbancy (at 670nm)
2.0
1.5
1.0
0.5
0
10
20
30
Amount of phenol (μg)

FIG. 3

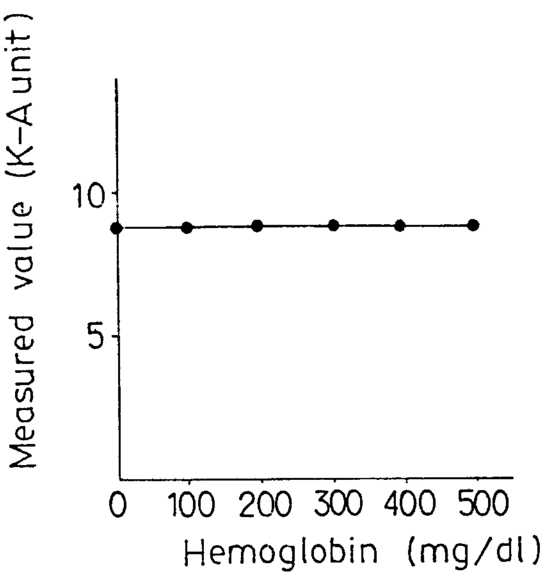


FIG. 4

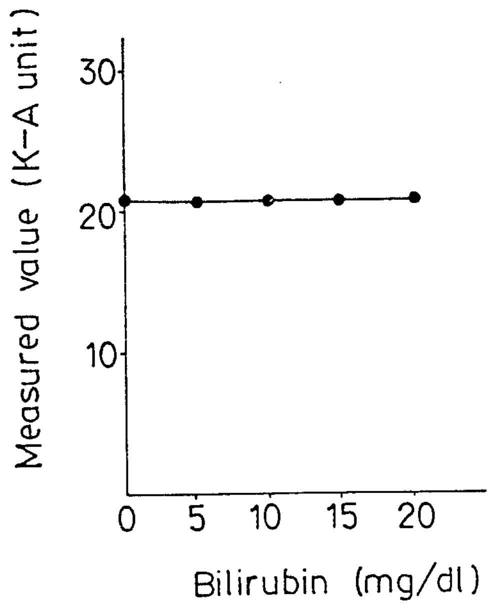


FIG. 5

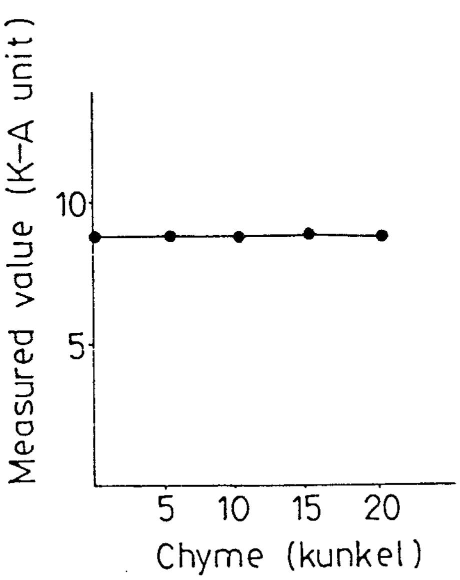


FIG. 6

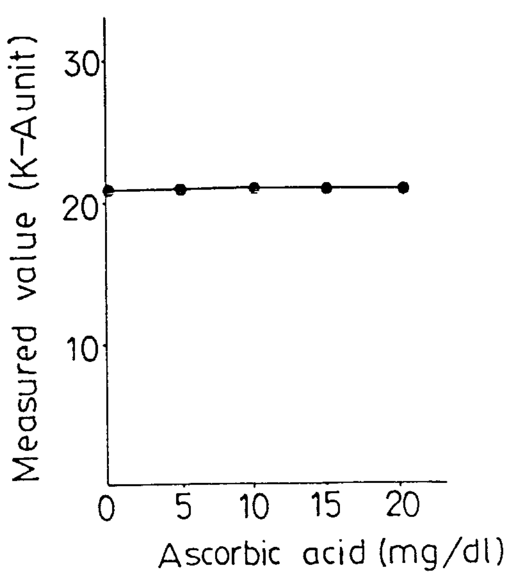

FIG. 7

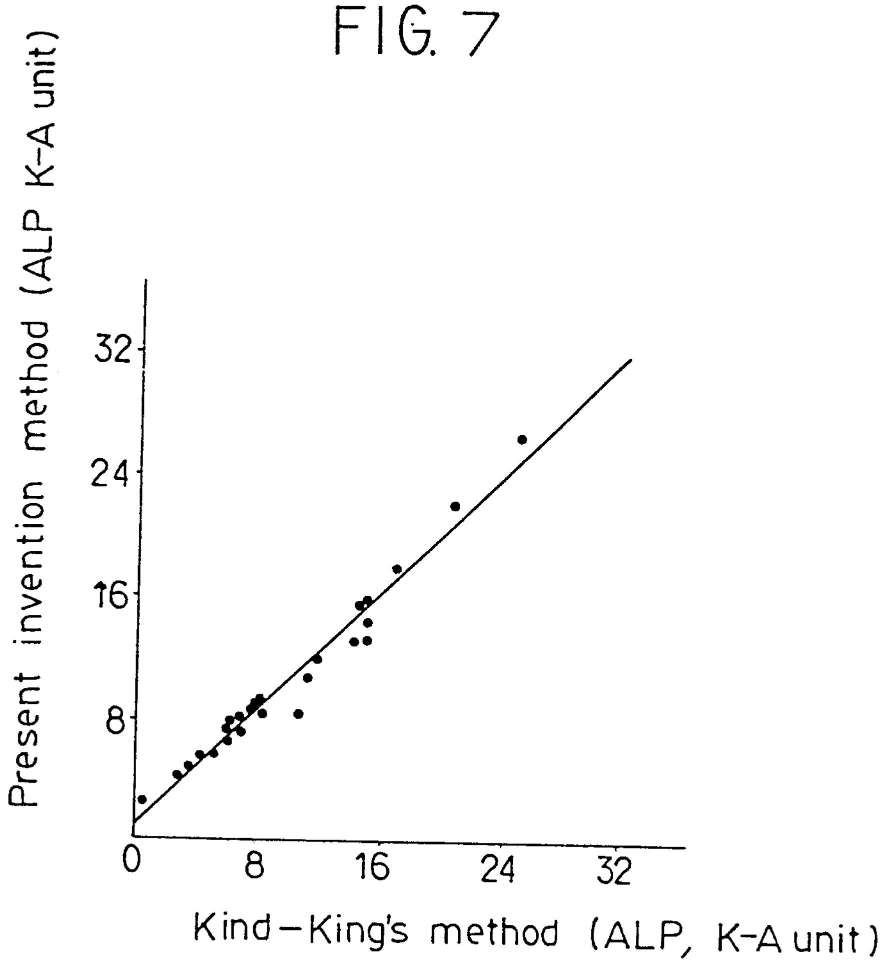


y=0.968x+1.04

r=0.9841

n=40